# EUROPÄISCHE PATENTSCHRIFT

(11) **EP 2 527 030 B1**
(45) Veröffentlichungstag und Bekanntmachung des Hinweises auf die Patenterteilung: **16.08.2017**
(21) Anmeldenummer: 12157121.0
(22) Anmeldetag: 27.02.2012
(51) Int. Cl.: B01F 7/24, B01F 15/02

(54) **Mischbehälter**
Mixing vessel
Récipient de mélange

(30) Priorität: 25.05.2011 DE 202011101177 U
(43) Veröffentlichungstag der Anmeldung: 28.11.2012
(73) Patentinhaber: Trioliet Holding B.V., 7575 BE Oldenzaal (NL)
(72) Erfinder: Liet, Cornelis Hendricus, 7581 PJ Losser (NL)
(74) Vertreter: Grünecker Patent- und Rechtsanwälte PartG mbB

(56) Entgegenhaltungen:
- WO-A1-95/13512
- WO-A2-2008/117096
- DE-A1- 3 629 486
- DE-A1-102005 021 381
- DE-U1-202007 017 166
- DE-U1-202008 004 251
- US-A- 4 765 747
- US-A- 5 240 321

## Beschreibung

Die Erfindung bezieht sich auf einen Mischbehälter der im Oberbegriff von Anspruch 1 bzw. 9 erläuterten Art.

Ein Mischbehälter ist aus der DE 102 06 270 A1 bekannt. Mischbehälter für Biogasanlagen sind der Biogasanlage vorgeschaltet und dienen dazu, die zu vergärende Biomasse aus unterschiedlichen Bestandteilen zu mischen, aufzulockern und dosiert in den Fermenter abzugeben. Die Mischbehälter sind bevorzugt stationär auf dem Dach bzw. einer Deckplatte oder neben dem Fermenter angeordnet oder mobil ausgebildet. In der Konstruktion sind derartige Mischbehälter für Biogasanlagen von der bewährten Konstruktion der Futtermischwagen abgeleitet. Dies bedeutet, die Mischbehälter weisen den eigentlichen Behälter auf, der einen sich nach oben erweiterten Querschnitt, eine obere Einfüllöffnung sowie eine einzige im Inneren des Behälters angeordnete, sich um eine vertikale Achse drehende, im Wesentlichen kegelförmig ausgebildete Mischschnecke und eine die Wandung des Behälters durchbrechenden Austragsöffnung aufweist. Beim bekannten Mischbehälter ist dieser Austragsöffnung eine Dosierschnecke zugeordnet, die anstelle der Schwerkraftförderung bei Futtermischwagen eine Zwangsförderung der Biomasse zum Fermenter übernimmt. Die Dosierschnecke ist so angeordnet, dass ihre Aufnahmeöffnung der Austrittsöffnung des Behälters entspricht; d.h. die Dosierschnecke befindet sich unmittelbar an der Außenseite des Behälters. Dadurch soll es möglich sein, auch schwieriges Fördergut (nass / klebrig / langfasrig) zu fördern. Beim bekannten Mischbehälter ist die Austragsöffnung und somit die Aufnahmeöffnung für die Dosierschnecke vollständig oder zumindest teilweise im Boden vorgesehen, und zwar an einer Stelle, die vom auf dem Boden projizierten Drehkreis der Mischschnecke überstrichen wird, d.h. die vorlaufende Kante der Mischschnecke läuft über die Aufnahmeöffnung der Dosierschnecke hinweg. Es hat sich jedoch herausgestellt, dass insbesondere diese Ausführung sehr stark anfällig für Verstopfungen ist, da sich beispielsweise langfasrige, klebrige Bestandteile der Biomasse zwischen der vorlaufenden Kante der Mischschnecke und der Kante der Aufnahmeöffnung/Austragsöffnung verklemmen und somit zu Verstopfungen führen. Dies tritt immer dann auf, auch bei den zum Teil in der Seitenwandung liegenden Aufnahmeöffnungen, wenn sich der Drehkreis der Mischschnecke mit der Aufnahmeöffnung überlappt.

Aus dem DE 20 2005 016 744 U1 ist ein Mischbehälter zum Beschicken von Biogasanlagen bekannt, bei dem Dosierschnecken einer sich durch die Seitenwandung erstreckenden Ausgabeöffnung, außerhalb des Behälters, zugeordnet sind. Hier muss, wie bei Futtermischern auch, der Förderdruck der Mischschnecke in Richtung der Seitenwand des Behälters dafür sorgen, dass die Biomasse durch den Förderdruck der Mischschnecke gegen die Seitenwandung durch die Ausgabeöffnung/Aufnahmeöffnung gedrückt wird. Dies ist jedoch bei der gegenüber von Futtermitteln doch unterschiedlichen Konsistenz der Biomasse, insbesondere wenn dieser als Koferment Stallmist zugesetzt wird, problematisch und nicht immer effektiv. Auch ist die Anordnung der Aufnahmeöffnung in der Seitenwand nur bei einer einzigen Mischschnecke im Behälter möglich.

Die DE 20 2004 002 546 U1 beschreibt einen Mischbehälter für Biogasanlagen mit einem Behälter und einer einzigen Mischschnecke sowie einer Austragsöffnung durch die Behälterwandung, die gleichzeitig die Aufnahmeöffnung für eine Dosierschnecke bildet. Der Mischbehälter wird mittels eines Ausschuborgans entleert, das die Biomasse an der Mischschnecke vorbei in Richtung auf die im Wesentlichen gegenüberliegende Austragsöffnung/Aufnahmeöffnung drückt. Diese Lösung, auch problematisch zu fördernde Biomasse mit hohem Durchsatz und dosiert auszutragen, ist jedoch relativ aufwändig.

Die DE 36 29 486 A1 offenbart eine Vorrichtung zum Mischen von feinkörnigem Gut, wie beispielsweise Zement, Kalk oder Asche bei dem das feinkörnige Gut in einem Mischbehälter mittels zwei oder drei durch Rohre ummantelten Mischschnecken mit unterschiedlicher Geschwindigkeit von unten nach oben gefördert wird und nach dem Verlassen der oberen Rohrenden mit gleicher Geschwindigkeit auf den Behälterboden zurückfällt. Mittels einer zwischen den Mischschnecken angeordneten Austragsschnecke kann das Gut aus dem Mischbehälter entnommen werden. Die Aufnahmeöffnung der Austragsschnecke befindet sich dabei oberhalb der auf den Behälterboden projizierten Drehkreise der Mischschnecken. Die bekannte Vorrichtung eignet sich allerdings nicht für Mischgut einer Biogasanlage.

Die DE 20 2007 017 166 U1 offenbart eine Kompakt-Biogasanlage mit einem Fermenter. Gattungsgemäße Mischbehälter für Biogasanlagen können beispielsweise einem derartigen Fermenter vorgeschaltet sein.

Weiter offenbart die DE 20 2008 004 251 U1 einen Fermentierbehälter zur Trockenfermentation.

Die US 5,240,321 offenbart einen Mischbehälter entsprechend den Oberbegriffen der Ansprüche 1 und 9 mit vertikal angeordneten Mischschnecken, der vorzugsweise bei der Abfallverarbeitung eingesetzt wird. Größere Bestandteile des Mischguts werden mit Klingen an den Mischschnecken in kleinere Stücke zerschnitten. Das so gemischte und zerkleinerte Material wird durch eine seitlich von allen Mischschnecken angeordnet Auslassöffnung abgegeben und mittels einer anschließenden Förderschnecke abgefördert.

Der Erfindung liegt die Aufgabe zugrunde, einen Mischbehälter für Biogasanlagen bereitzustellen, der auch problematisches Mischgut sicher und problemlos ausgeben kann.

Die Aufgabe wird durch die im Anspruch 1 angegebenen Merkmale gelöst.

Durch die erfindungsgemäße Ausgestaltung wird die Dosierschnecke zum Austragen des Mischgutes aus dem Mischbehälter an einer Stelle angeordnet, die ein mehr oder weniger großes Hindernis für den konstruktiv berechneten Mischgutstrom, wie er von den Mischschnecken im Inneren des Behälters erzeugt wird, bildet. Es sei daran erinnert, dass die Innenoberfläche der Wandung des Behälters, sowohl im Bereich der Seitenwandung als auch im Bereich der Arbeitsflächen auf eine optimale Mischbewegung in Verbindung mit der speziellen Konstruktion und Form der Mischschnecken ausgelegt ist. Da jedoch die Aufnahmeöffnung für die Dosierschnecke außerhalb der Drehkreise angeordnet ist, wird eine Verstopfung, beispielsweise durch Festklemmen von langfasrigem Mischgut, wirksam verhindert.

Vorteilhafte Weiterbildungen der Erfindung sind den Unteransprüchen zu entnehmen.

Die Erfindung eignet sich besonders für Mischbehälter mit insbesondere genau zwei Mischschnecken, da dadurch die Dosierschnecke mit ihrer Aufnahmeöffnung zwischen den Mischschnecken angeordnet werden kann.

Weiterhin ist es besonders vorteilhaft, wenn wenigstens ein Teil der Dosierschnecke aus der Aufnahmeöffnung heraus in den Innenraum des Behälters vorsteht. Auf diese Weise wird die Hinderniswirkung der Dosierschnecke und somit ihre Aufnahmewirkung verbessert.

Dabei ist die Ausrichtung der Aufnahmeöffnung in Axialrichtung der Drehachse der Dosierschnecke und/oder ihre Erstreckung über wenigstens eine Schneckenwindung besonders vorteilhaft.

Die Aufnahmeöffnung kann im Wesentlichen vertikal ausgerichtet sein, wobei auch eine vollständig im Innenraum des Behälters freiliegende Dosierschnecke mit umfasst werden kann. Bei dieser Ausrichtung bietet sich besonders die Anordnung der Aufnahmeöffnung in einem Zwickeleinbau zwischen benachbarten Mischschnecken oder anstelle eines Zwickeleinbaus an.

Die Aufnahmeöffnung kann weiterhin horizontal ausgerichtet sein, wobei auch eine vollständig freiliegende Dosierschnecke mit umfasst werden soll.

Bei dieser Art der Aufnahmeöffnung bietet sich die Anordnung der Dosierschnecke mit ihrer Aufnahmeöffnung in einen horizontalen Abstand zwischen zwei benachbarten Drehkreisen an.

Eine weitere Möglichkeit, die sich insbesondere dann anbietet, wenn die Mischschnecken vertikal versetzt zueinander im Behälter angeordnet sind, ist die Anordnung der Aufnahmeöffnung in die durch den vertikalen Abstand zwischen den Drehkreisen gebildeten Stufe.

Dabei muss die Dosierschnecke nicht unbedingt im Inneren des Behälters angeordnet sein, wenn sich die Drehkreise im horizontalen und/oder vertikalen Abstand zueinander befinden und die Aufnahmeöffnung in diesem Abstand angeordnet ist.

Ferner wird die Aufgabenstellung bei einem Mischbehälter mit den Merkmalen des Oberbegriffs von Anspruch 9 mit den Merkmalen des kennzeichnenden Teils gelöst, gemäß dem die Dosierschnecke eine Aufnahmeöffnung für das Mischgut aufweist, die sich zwischen den in einem horizontalen und/oder vertikalen Abstand zueinander angeordneten Drehkreisen der Mischschnecken befindet.

Vorteilhafte Ausführungsformen der Erfindung werden nachfolgend anhand der Zeichnungen näher erläutert. Es zeigen:
- Fig. 1: ein erstes Ausführungsbeispiel eines erfindungsgemäßen Mischbehälters in verschiedenen Darstellungen, wobei Fig. 1A eine perspektivische Draufsicht, Fig. 1 B eine Draufsicht, Fig. 1C den Schnitt A - A aus Fig. 1B und Fig. 1D den Schnitt B-B aus Fig. 1B zeigen,
- Fig. 2: ein weiteres Ausführungsbeispiel des erfindungsgemäßen Mischbehälters, wobei Fig. 2A eine perspektivische Draufsicht, Fig. 2B eine Draufsicht, Fig. 2C den Schnitt B - B aus Fig. 2B und Fig. 2D den Schnitt C - C aus Fig. 2B zeigt,
- Fig. 3: ein weiteres Ausführungsbeispiel eines erfindungsgemäßen Mischbehälters, wobei Fig. 3A eine perspektivische Draufsicht, Fig. 3B eine Draufsicht, Fig. 3C den Schnitt A - A aus Fig. 3B und Fig. 3D den Schnitt B - B aus Fig. 3B zeigt,
- Fig. 4: ein weiteres Ausführungsbeispiel eines erfindungsgemäßen Mischbehälters, wobei Fig. 4A eine perspektivische Draufsicht, Fig. 4B eine Draufsicht, Fig. 4C den Schnitt A - A aus Fig. 4B und Fig. 4D den Schnitt B - B aus Fig. 4B zeigt, und
- Fig. 5: ein weiteres Ausführungsbeispiel eines erfindungsgemäßen Mischbehälters, wobei Fig. 5A eine perspektivische Draufsicht, Fig. 5B eine Draufsicht, Fig. 5C den Schnitt B - B aus Fig. 5B und Fig. 5D den Schnitt A - A aus Fig. 5B zeigt.

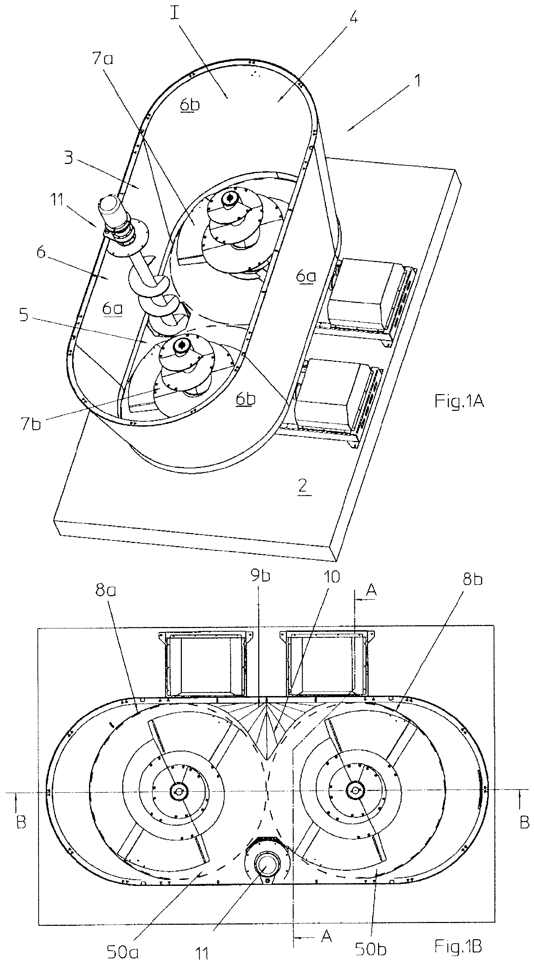

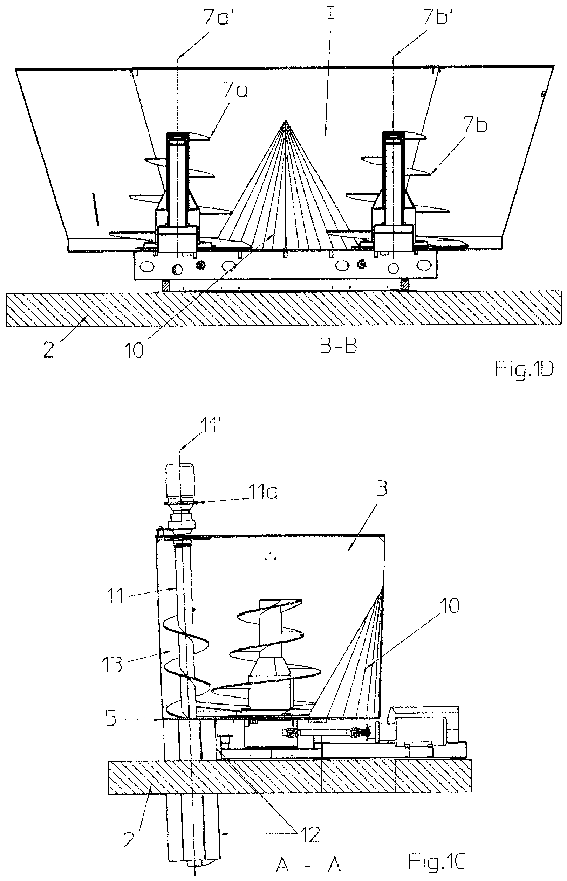

Fig. 1 zeigt ein erstes Ausführungsbeispiel eines erfindungsgemäßen Mischbehälters 1, der insbesondere zum Herstellen von Mischungen für Biogasanlagen ausgebildet ist, dessen Konstruktion jedoch von der Konstruktion üblicher Futtermischbehälter angeleitet ist. Der Mischbehälter 1 sitzt hier auf einem Dach oder einer Deckplatte 2 eines nicht dargestellten Fermenters. Der Mischbehälter 1 enthält den eigentlichen Behälter 3, der als nach oben über eine Einfüllöffnung 4 beschickbare Wanne ausgebildet ist und einen Boden 5 sowie eine den Innenraum I umschließende Seitenwandung 6 aufweist. Die Seitenwandung 6 enthält im dargestellten Ausführungsbeispiel längere Seiten 6a und kürzere Seiten 6b, wobei sich die längeren Seiten 6a im Wesentlichen senkrecht vom Boden 5 zur Einfüllöffnung 4 erstrecken und die kürzeren Seiten 6b nach außen ausgerundet und sich zum Vergrößern des Fassungsvermögens nach oben trichterartig erweiternd ausgebildet sind, so dass die Bodenfläche des Bodens 5 kleiner ist als die Fläche der Einfüllöffnung 4. Die Erfindung ist jedoch auch für andere Behälterformen einsetzbar. Im Inneren des Behälters 3 sind zwei der üblichen Mischschnecken 7a, 7b vorgesehen, die jeweils um im Wesentlichen vertikal zum Boden 5 verlaufende Drehachsen 7a' und 7b' drehend angetrieben werden und jeweils unterhalb der Mischschnecke 7a, 7b liegende Arbeitsflächen 50a, 50b überstreichen. Im dargestellten Ausführungsbeispiel liegen die Arbeitsflächen 50a, 50b und somit die sich bei der Drehung durch Projektion auf die Arbeitsflächen 50a, 50b ergebenden Drehkreise 8a, 8b (Fig. 1 B) auf dem Boden 5. Damit liegen die Drehkreise 8a, 8b in der gleichen Ebene und sind so nahe wie möglich zueinander angeordnet (d.h. die Drehachsen 7a' und 7b' weisen den geringst möglichen Abstand zueinander auf, so dass sich die Schnecken bei ihrer Drehbewegung gerade nicht berühren). In den seitlich davon angeordneten Zwickelbereichen 9a, 9b zu den längeren Seiten 6a ist auf einer Seite ein Zwickeleinbau 10 angeordnet, der den Totraum zwischen den Drehkreisen 8a und 8b überdeckt, so dass sich dort kein Mischgut ansammeln kann. Die gegenüberliegende Seite weist keinen Zwickeleinbau auf, vielmehr befindet sich dort eine Dosierschnecke 11, die aus ihrer im Innenraum I des Behälters 3 freiliegenden Anordnung durch den Boden 5 und durch eine Öffnung im Dach 2 in den nicht gezeigten Fermenter hineinragt. Die Dosierschnecke 11 ist von üblicher Ausgestaltung und um eine hier im Wesentlichen senkrecht angeordnete Achse 11' über einen eigenen Motor 11 a antreibbar. Der Antrieb ist bevorzugt in beiden Drehrichtungen möglich, um das Mischen nicht zu behindern. Dadurch kann ein Festsetzen von Mischgut verhindert werden, was in einer stillstehenden Dosierschnecke möglich wäre. Auch das vorlaufende Ende der Dosierschnecke ist so ausgebildet, dass es nicht zu Problemen kommt.

Die Dosierschnecke 11 ist oberhalb des Bodens 5 mit einer Aufnahmeöffnung 13 versehen, die sich im dargestellten Ausführungsbeispiel über den gesamten Bereich oberhalb des Bodens erstreckt, d.h. oberhalb des Bodens 5 enthält die Dosierschnecke kein Gehäuse und liegt mit wenigstens einer Schneckenwindung frei. Unterhalb des Bodens 5 ist ein Gehäuse 12 vorhanden.

Diese Ausgestaltung gestattet es der Dosierschnecke, Mischgut aus dem Behälter 3 abzuziehen, das aus dem Bewegungsbereich des Mischgutes durch die Mischschnecken stammt, ohne dass die Mischschnecken eine aktive Rolle beim Herauspressen oder Abstreifen des Mischguts durch eine Öffnung zur Dosierschnecke zu übernehmen haben. Vielmehr bedient sich die Dosierschnecke am Mischgut, das durch die Mischschnecken im Zwickelbereich 9a abgelagert wird. Dadurch wirkt lediglich die Förderbewegung der Dosierschnecke auf das Mischgut ein und zieht dieses problemlos nach außen. Auf diese Weise ist es problemlos möglich, selbst stark klebriges und langfasriges Mischgut, beispielsweise wenn strohhaltiger Stallmist oder angerottetes, nasses, langfasriges Gras als Koferment eingesetzt wird, ohne die Gefahr von Verstopfungen zu fördern.

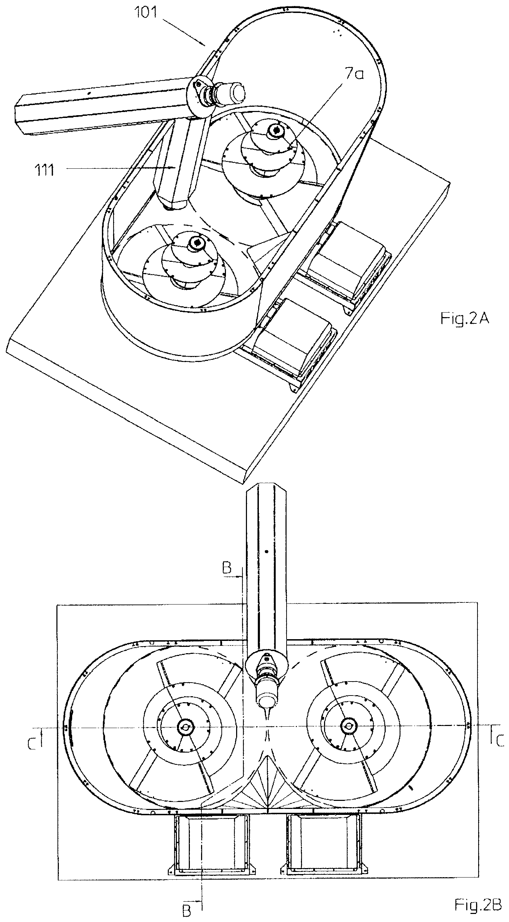

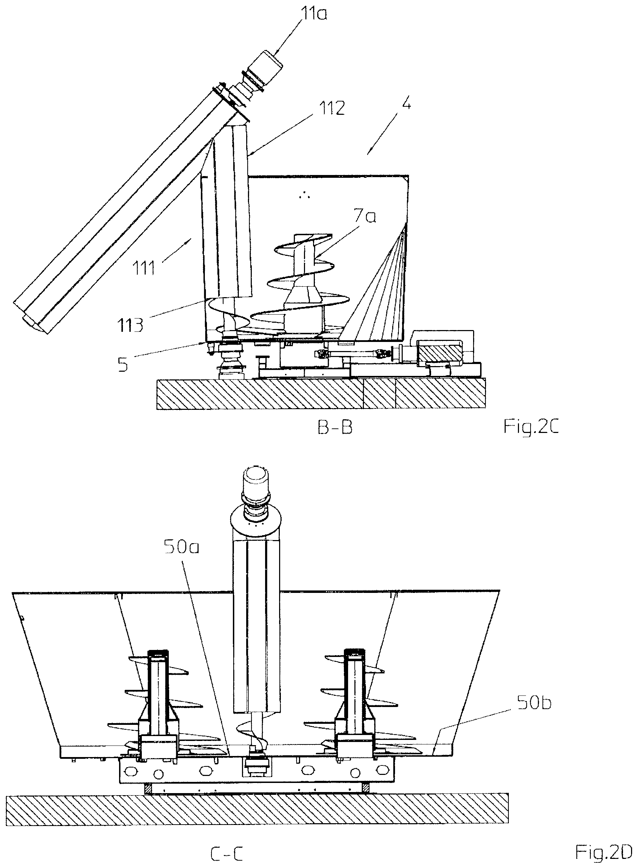

Fig. 2 zeigt ein weiteres Ausführungsbeispiel eines erfindungsgemäß ausgerüsteten Mischbehälters 101, der sich vom Mischbehälter 1 nur durch die nachfolgend beschriebenen Einzelheiten unterscheidet.

Der Mischbehälter 101 unterscheidet sich vom Mischbehälter 1 durch eine abgewandelte Dosierschnecke 111, die hier nach oben, d.h. aus der Einfüllöffnung 4 heraus fördert. Darüber hinaus ist die Dosierschnecke 111 lediglich im unteren, d.h. sich mit mehreren Schneckenwindungen vom Boden 5 bis knapp unterhalb der Spitze der Mischschnecke 7a erstreckenden Bereich ringsum zur Ausbildung einer Aufnahmeöffnung 113 offen, während der darüberliegende Bereich durch ein Gehäuse 112 verschlossen ist.

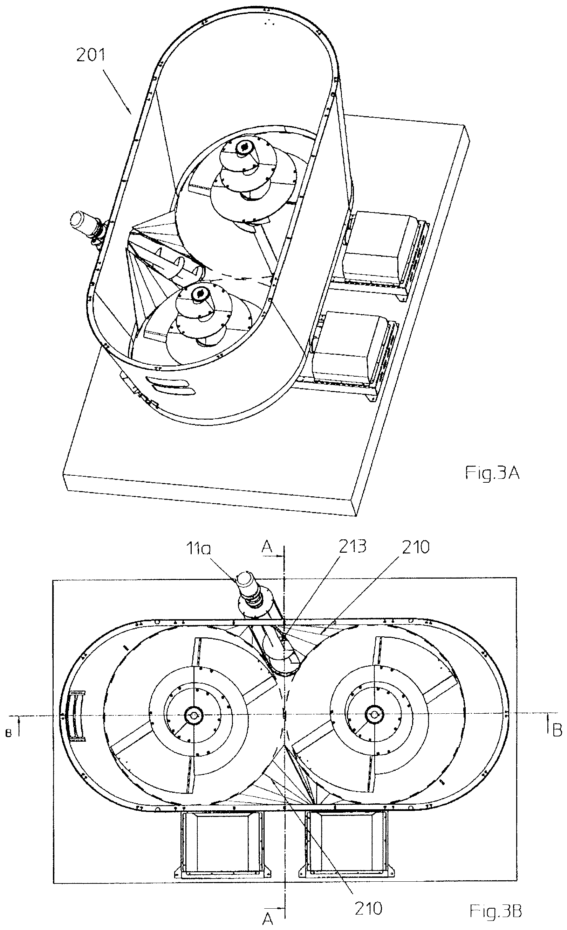

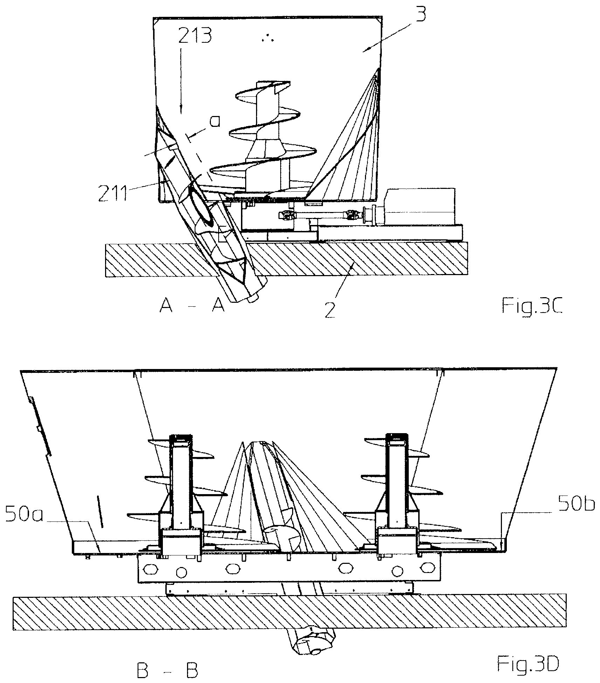

Fig. 3 zeigt ein weiteres Ausführungsbeispiel eines erfindungsgemäßen Mischbehälters 201, der sich von den vorangegangenen Ausführungsbeispielen nur durch die nachfolgend beschriebenen Einzelheiten unterscheidet. Der Mischbehälter 201 unterscheidet sich durch das Vorhandensein zweier Zwickeleinbauten 210, wobei in einem der Zwickeleinbauten eine sich wiederum über wenigstens eine, bevorzugt mehrere Schneckenwindungen erstreckende Aufnahmeöffnung 213 angeordnet ist, die sich in Axialrichtung zur Dosierschnecke 211 erstreckt und so ausgebildet ist, dass die Dosierschnecke 211 über den Bereich a in den Innenraum des Behälters 3 vorsteht (siehe Fig. 3C). Die Dosierschnecke 211 fördert wieder nach unten durch den Deckel 2 direkt in den Fermenter.

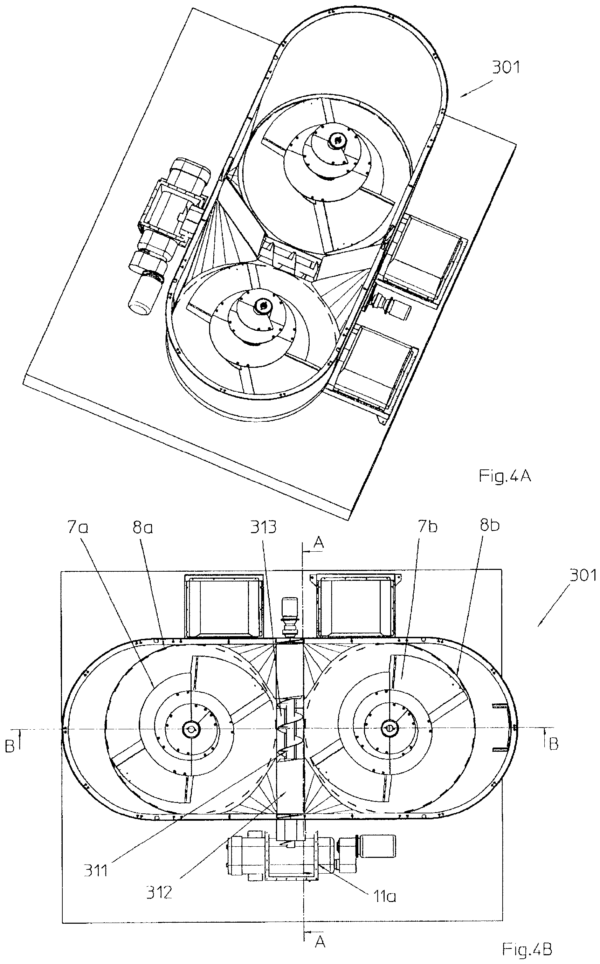

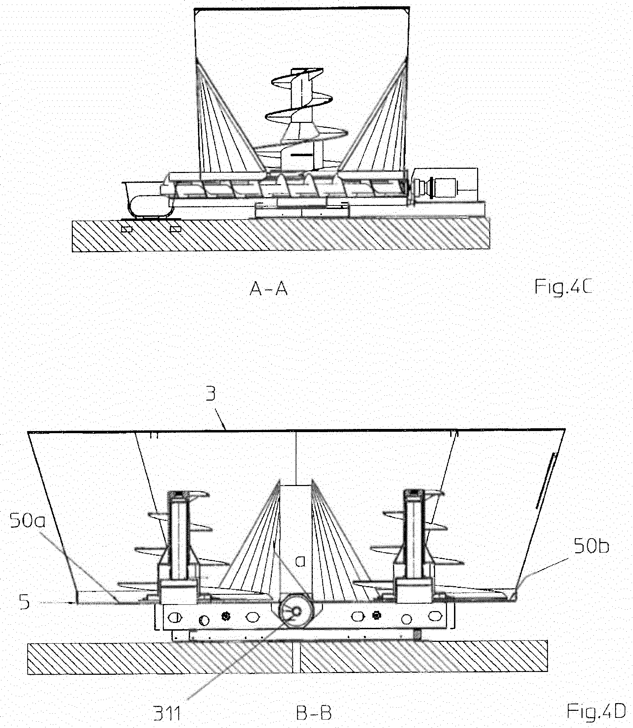

Fig. 4 zeigt ein weiteres Ausführungsbeispiel eines Mischbehälters 301, wobei nur die gegenüber den vorangegangenen Ausführungsbeispielen veränderten Einzelheiten dargestellt und erläutert werden sollen.

Der Mischbehälter 301 unterscheidet sich von den vorangegangenen Ausführungsbeispielen durch einen vergrößerten horizontalen Abstand der Mischschnecken 7a, 7b derart, dass ihre Drehkreise 8a, 8b auch im Bereich ihrer größten Annäherung einen Abstand aufweisen. Der Abstand ist so bemessen, dass dort eine Aufnahmeöffnung 313 vorgesehen werden kann, die im Wesentlichen horizontal ausgerichtet ist und sich zwischen den Arbeitsflächen 50a, 50b erstreckt und über wenigstens eine, bevorzugt mehrere Schneckenwindungen reicht. Die Aufnahmeöffnung 313 ist rechteckig und langgestreckt und symmetrisch zum Bereich der größten Annäherung der Drehkreise 8a, 8b angeordnet. Die Dosierschnecke 311 dreht sich um eine horizontale und im Wesentlichen quer zwischen den langen Seitenwänden 6a verlaufenden Drehachse 11' und wird wiederum durch einen eigenen Motor 11a angetrieben. Die Aufnahmeöffnung ist vorzugsweise so gelegt, dass die Dosierschnecke 311 um den Bereich a aus der Aufnahmeöffnung 313 in den Innenraum des Behälters 3 vorsteht. Die Dosierschnecke 311 mit ihrer Aufnahmeöffnung 313 befindet sich somit im Zwischenraum zwischen den Drehkreisen der Mischschnecken, so dass die Mischschnecken keine Einschiebearbeit in die Aufnahmeöffnung 313 leisten müssen, sondern das Mischgut durch die Bewegung der Dosierschnecke aus der Aufnahmeöffnung 313 gezogen wird. Eine Verstopfungsgefahr durch langfasriges Gut, das zwischen der Mischschnecke und der Aufnahmeöffnung festgeklemmt wird, besteht somit nicht.

Bei nicht dargestellten Abwandlungen dieses Ausführungsbeispiels könnte die Dosierschnecke auch oberhalb des Bodens angeordnet sein und bevorzugt gehäuselos oder nur partiell mit einem Gehäuse versehen sein. Auch eine vollständig unter Bodenniveau versenkte Anordnung der Dosierschnecke ist möglich.

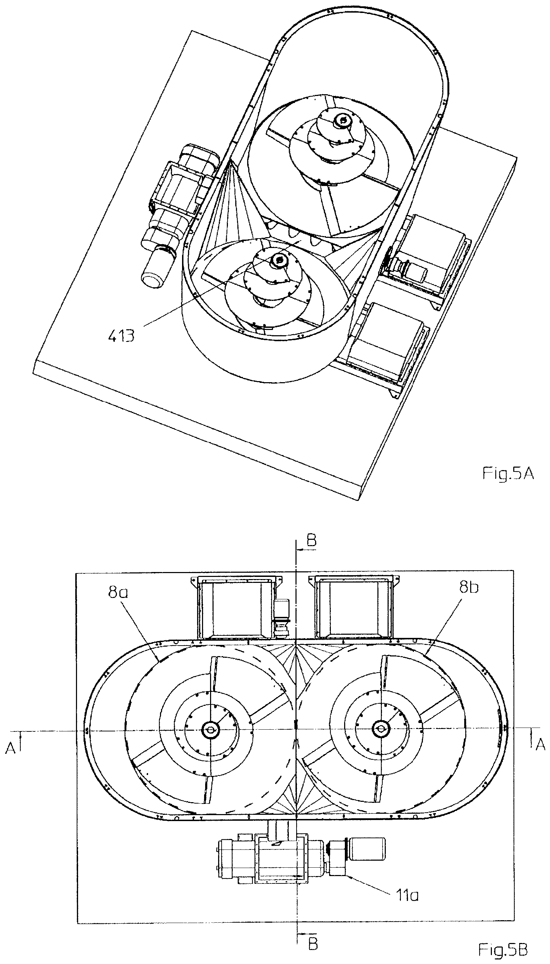

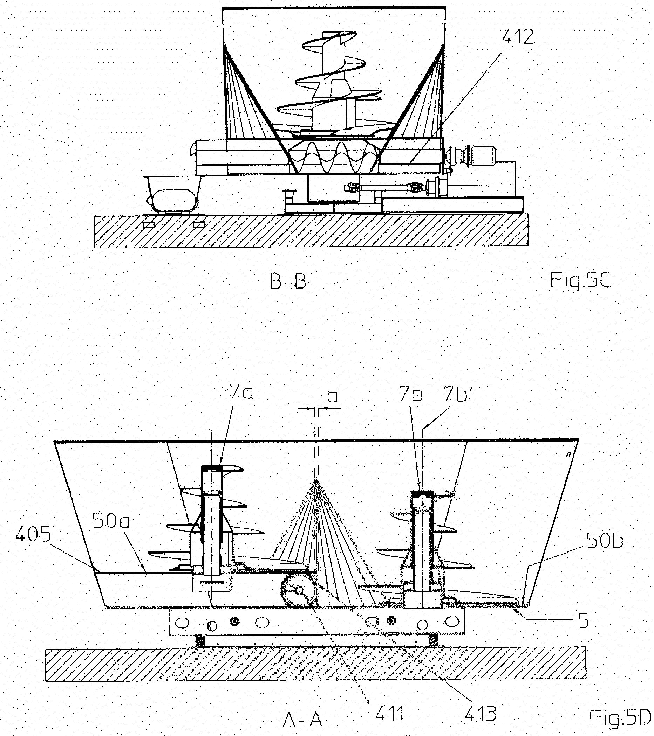

Ein weiteres Ausführungsbeispiel eines Mischbehälters 401 mit horizontal liegender Dosierschnecke 411 zeigt Fig. 5. Der Mischbehälter 401 enthält zwei der üblichen Mischschnecken 7a, 7b, die im Abstand zueinander auf Arbeitsflächen 50a, 50b in unterschiedlichen Höhenlagen angeordnet sind. Dabei befindet sich die untere Arbeitsfläche 50b direkt auf dem Boden 5 und die obere Arbeitsfläche 50a auf einem Podest 405 im Abstand zum Boden 5. Der vertikale Unterschied zwischen den Arbeitsflächen 50a, 50b wird durch eine Stufe gebildet, die direkt im Bereich größter Annäherung (fast Berührung) der Drehkreise 8a, 8b verläuft. In der Stufe befindet sich die langgestreckt rechteckige Aufnahmeöffnung 413 über bevorzugt mehrere Windungen der Dosierschnecke 411, derart, dass die Aufnahmeöffnung 413 nicht von den Drehkreisen 8a, 8b der Mischschnecken 7a, 7b überstrichen wird. Die Dosierschnecke 411 steht bevorzugt wiederum um den Bereich a aus der Aufnahmeöffnung 413 vor, wobei der Bereich a so gewählt ist, dass die vorlaufende Kante der Mischschnecke 7b, die den größten Abstand zur Drehachse 7b' aufweist, unter der Dosierschnecke 11, d.h. im Zwickel, den die Rundung der Dosierschnecke 411 mit dem Boden 5 bildet, hindurchtauchen kann. Die Höhenstaffelung der Arbeitsflächen 50a, 50b kann auch über eine direkt im Boden 5 vorgesehene Stufe erreicht werden.

In Abwandlung der beschriebenen und gezeichneten Ausführungsbeispiele sind auch andere Anordnungen der Dosierschnecke möglich. Die Erfindung kann auch bei Mischbehältern mit mehr als zwei Mischschnecken angewandt werden (mehrere Dosierschnecken).

## Patentansprüche

1. Mischbehälter für eine Biogasanlage (1, 101, 201), mit einem Behälter (3) mit einer einen Innenraum (I) umschließenden Seitenwandung (6), in dem wenigstens zwei Mischschnecken (7a, 7b) angeordnet sind, die jeweils einen auf eine Arbeitsfläche (50a, 50b) projizierten Drehkreis (8a, 8b) überstreichen, und mit einer Dosierschnecke (11, 111, 211) zum Austragen des Mischguts, wobei die Dosierschnecke eine Aufnahmeöffnung (13, 113, 213) für das Mischgut aufweist, die sich außerhalb der Drehkreise (8a, 8b) befindet, **dadurch gekennzeichnet, dass** die Dosierschnecke im Innenraum (I) des Behälters (3) angeordnet ist und dass sich die Aufnahmeöffnung (13, 113, 213) in einem durch zwei benachbarten Drehkreise (8a, 8b) gebildeten Zwickelbereich und/oder in einem Zwickeleinbau (210) befindet.

2. Mischbehälter nach Anspruch 1, **dadurch gekennzeichnet, dass** sich die Aufnahmeöffnung (13, 113, 213, 313, 413) zwischen den Drehkreisen (8a, 8b) der Mischschnecken (7a, 7b) befindet.

3. Mischbehälter nach Anspruch 1, **dadurch gekennzeichnet, dass** wenigstens ein Teil der Dosierschnecke (11, 111, 211, 311, 411) aus der Aufnahmeöffnung (13, 113, 213, 313, 413) in den Innenraum (I) vorsteht.

4. Mischbehälter nach einem der Ansprüche 1 bis 3, **dadurch gekennzeichnet, dass** sich die Aufnahmeöffnung (13, 113, 213, 313, 413) in Axialrichtung der Drehachse (11') der Dosierschnecke (11, 111, 211, 311, 411) erstreckt.

5. Mischbehälter nach einem der Ansprüche 1 bis 4, **dadurch gekennzeichnet, dass** sich die Aufnahmeöffnung (13, 113, 213, 313, 413) über wenigstens eine Schneckenwindung der Dosierschnecke erstreckt.

6. Mischbehälter nach einem der Ansprüche 1 bis 5, **dadurch gekennzeichnet, dass** sich die Aufnahmeöffnung (313, 413) zwischen den in einem horizontalen und/oder vertikalen Abstand zueinander angeordneten Drehkreisen (8a, 8b) der Mischschnecken (7a, 7b) befindet.

7. Mischbehälter nach einem der Ansprüche 1 bis 6, **dadurch gekennzeichnet, dass** die Aufnahmeöffnung (13, 113, 213) im Wesentlichen vertikal ausgerichtet ist.

8. Mischbehälter nach einem der Ansprüche 1 bis 6, **dadurch gekennzeichnet, dass** die Aufnahmeöffnung (313, 413) im Wesentlichen horizontal ausgerichtet ist.

9. Mischbehälter für eine Biogasanlage (301, 401), mit einem Behälter (3) mit einer einen Innenraum (I) umschließenden Seitenwandung (6), in dem wenigstens zwei Mischschnecken (7a, 7b) angeordnet sind, die jeweils einen auf eine Arbeitsfläche (50a, 50b) projizierten Drehkreis (8a, 8b) überstreichen, und mit einer Dosierschnecke (311, 411) zum Austragen des Mischguts, wobei die Dosierschnecke eine Aufnahmeöffnung (313, 413) für das Mischgut aufweist, **dadurch gekennzeichnet, dass** die Aufnahmeöffnung sich zwischen den in einem horizontalen und/oder vertikalen Abstand zueinander angeordneten Drehkreisen (8a, 8b) der Mischschnecken (7a, 7b) befindet.

## Claims

1. Mixing vessel for a biogas plant (1, 101, 201) comprising a container (3) with a side wall (6) enclosing an interior (1) in which at least two mixing screws (7a, 7b) are disposed, each of which sweeps a turning circle (8a, 8b) projected onto a working surface (50a, 50b), and comprising a metering screw (11, 111, 211) for discharging the mix, the metering screw having an intake opening (13, 113, 213) for the mix which is located outside the turning circles (8a, 8b), **characterised in that** the metering screw is disposed in the interior (1) of the container (3) and the intake opening (13, 113, 213) is located in a spandrel area formed by two adjacent turning circles (8a, 8b) and/or in an integrated spandrel fitting (210).

2. Mixing vessel as claimed in claim 1, **characterised in that** the intake opening (13, 113, 213, 313, 413) is located between the turning circles (8a, 8b) of the mixing screws (7a, 7b).

3. Mixing vessel as claimed in claim 1, **characterised in that** at least a part of the metering screw (11, 111, 211, 311, 411) projects out of the intake opening (13, 113, 213, 313, 413) into the interior (1).

4. Mixing vessel as claimed in one of claims 1 to 3, **characterised in that** the intake opening (13, 113, 213, 313, 413) extends in the axial direction of the axis of rotation (11') of the metering screw (11, 111, 211, 311, 411) .

5. Mixing vessel as claimed in one of claims 1 to 4, **characterised in that** the intake opening (13, 113, 213, 313, 413) extends across at least one screw turn of the metering screw.

6. Mixing vessel as claimed in one of claims 1 to 5, **characterised in that** the intake opening (313, 413) is located between the turning circles (8a, 8b) of the mixing screws (7a, 7b) disposed at a horizontal and/or vertical distance from one another.

7. Mixing vessel as claimed in one of claims 1 to 6, **characterised in that** the intake opening (13, 113, 213) is oriented substantially vertically.

8. Mixing vessel as claimed in one of claims 1 to 6, **characterised in that** the intake opening (313, 413) is oriented substantially horizontally.

9. Mixing vessel for a biogas plant (301, 401), comprising a container (3) with a side wall (6) enclosing an interior (1) in which at least two mixing screws (7a, 7b) are disposed, each of which sweeps a turning circle (8a, 8b) projected onto a working surface (50a, 50b), and comprising a metering screw (311, 411) for discharging the mix, the metering screw having an intake opening (313, 413) for the mix, **characterised in that** the intake opening is located between the turning circles (8a, 8b) of the mixing screws (7a, 7b) disposed at a horizontal and/or vertical distance from one another.

## Revendications

1. Réservoir de mélange pour une installation de production de biogaz (1, 101, 201), comprenant un réservoir (3) avec un ensemble de paroi latérale (6) entourant un espace intérieur (I) dans lequel sont agencées au moins deux vis sans fin de mélange (7a, 7b), qui balayent chacun respectivement un cercle de rotation (8a, 8b) en projection sur une surface de travail (50a, 50b), l'ensemble comprenant également une vis sans fin de dosage (11, 111, 211) pour extraire le produit mélangé, et la vis sans fin de dosage présentant une ouverture de réception (13, 113, 213) pour le produit mélangé, qui se trouve à l'extérieur desdits cercles de rotation (8a, 8b),
**caractérisé en ce que** la vis sans fin de dosage est agencée dans l'espace intérieur (I) du réservoir (3), et **en ce que** l'ouverture de réception (3, 113, 213) se trouve dans une zone cunéiforme formée par les deux cercles de rotation (8a, 8b) et/ou dans un insert de forme cunéiforme (210).

2. Réservoir de mélange selon la revendication 1, **caractérisé en ce que** l'ouverture de réception (13, 113, 213, 313, 413) se trouve entre les cercles de rotation (8a, 8b) des vis sans fin de mélange (7a, 7b).

3. Réservoir de mélange selon la revendication 1, **caractérisé en ce qu'**au moins une partie de la vis sans fin de dosage (11, 111, 211, 311, 411) fait saillie hors de l'ouverture de réception (13, 113, 213, 313, 413), dans l'espace intérieur (I).

4. Réservoir de mélange selon l'une des revendications 1 à 3, **caractérisé en ce que** l'ouverture de réception (13, 113, 213, 313, 413) s'étend dans la direction axiale de l'axe de rotation (11') de la vis sans fin de dosage (11, 111, 211, 311, 411).

5. Réservoir de mélange selon l'une des revendications 1 à 4, **caractérisé en ce que** l'ouverture de réception (13, 113, 213, 313, 413) s'étend sur au moins une spire de vis sans fin de la vis sans fin de dosage.

6. Réservoir de mélange selon l'une des revendications 1 à 5, **caractérisé en ce que** l'ouverture de réception (313, 413) se trouve entre les cercles de rotation (8a, 8b) des vis sans fin de mélange (7a, 7b), qui sont agencés à distance horizontale et/ou verticale l'un de l'autre.

7. Réservoir de mélange selon l'une des revendications 1 à 6, **caractérisé en ce que** l'ouverture de réception (13, 113, 213) est d'une orientation essentiellement verticale.

8. Réservoir de mélange selon l'une des revendications 1 à 6, **caractérisé en ce que** l'ouverture de réception (313, 413) est d'une orientation essentiellement horizontale.

9. Réservoir de mélange pour une installation de production de biogaz (301, 401), comprenant un réservoir (3) avec un ensemble de paroi latérale (6) entourant un espace intérieur (I) dans lequel sont agencées au moins deux vis sans fin de mélange (7a, 7b), qui balayent chacun respectivement un cercle de rotation (8a, 8b) en projection sur une surface de travail (50a, 50b), l'ensemble comprenant également une vis sans fin de dosage (311, 411) pour extraire le produit mélangé, et la vis sans fin de dosage présentant une ouverture de réception (313, 413) pour le produit mélangé,
**caractérisé en ce que** l'ouverture de réception se trouve entre les cercles de rotation (8a, 8b) des vis sans fin de mélange (7a, 7b), qui sont agencés à distance horizontale et/ou verticale l'un de l'autre.
